# EUROPÄISCHE PATENTSCHRIFT

(11) **EP 2 548 564 B1**
(45) Veröffentlichungstag und Bekanntmachung des Hinweises auf die Patenterteilung: **18.06.2014**
(21) Anmeldenummer: 12176150.6
(22) Anmeldetag: 12.07.2012
(51) Int. Cl.: A61K 36/03, A61K 36/185, A61K 36/71, A61K 36/81, A61K 36/72, A61P 3/04

(54) **Pharmazeutisches Mittel, Ernährung für einen Patienten sowie Verfahren zur Gewichtsreduzierung**
Pharmaceutical agent, nutrition for a patient and weight reduction method
Produit pharmaceutique, alimentation d'un patient et procédé de réduction du poids

(30) Priorität: 19.07.2011 DE 202011050757 U; 19.07.2011 US 201113185590
(43) Veröffentlichungstag der Anmeldung: 23.01.2013
(73) Patentinhaber: Schnappinger, Uta, 27632 Midlum (DE)
(72) Erfinder: Schnappinger, Uta, 27632 Midlum (DE)
(74) Vertreter: Hansen, Jochen

(56) Entgegenhaltungen:
- BE-A7- 1 005 963
- MX-A- PA06 010 173
- DATABASE WPI Week 200053 Thomson Scientific, London, GB; AN 2000-570885 XP002683149, & RU 2 145 866 C1 (DOKTOR N CO LTD) 27. Februar 2000 (2000-02-27)
- DATABASE EPODOC [Online] EUROPEAN PATENT OFFICE, THE HAGUE, NL; 20. Juni 2008 (2008-06-20), Byun Moo Won: "Composition for prevention of obesity containing a hot pepper extract and health supporting foods containing the same", XP002683285, Database accession no. KR-20070121865-A & KR 2008 0048426 A (BYUN MOO WON [KR]) 2. Juni 2008 (2008-06-02)
- DATABASE WPI Week 200380 Thomson Scientific, London, GB; AN 2003-861957 XP002683154, & KR 2003 0060157 A (KWANG DONG PHARM CO LTD) 16. Juli 2003 (2003-07-16)
- WEISER M ET AL: "Adjuvant therapy of cardiac diseases with Cralonin", BIOLOGISCHE MEDIZIN 2000 DE, Bd. 29, Nr. 2, 2000, Seiten 72-79, XP009162691, ISSN: 0340-8671

## Beschreibung

Die Erfindung betrifft ein homöopathisches Arzneimittel zur Verwendung zur Gewichtsreduzierung, umfassend die folgenden Bestandteile Adonis vernalis, Calcium carbonicum Hahnemann, Capsicum, Cascara, Fucus vesiculosus, Graphites und Kalium Carbonicum sowie Phytolacca.

Die Homöopathie ist eine Form der alternativen Medizin. Ärzte oder Homöopathen behandeln Patienten mit stark verdünnten Präparationen, pharmazeutischen Mitteln, die bei gesunden Menschen die Symptome auslösen, die denen der zu behandelnden Patienten ähneln. Mittels dieser Behandlung sollen die Ursachen der Symptome behandelt werden. Im Rahmen der Homöopathie wird der Begriff Mittel für eine Substanz verwendet, die mit einem bestimmten Verfahren hergestellt worden ist und für Patienten Verwendung findet.

Das Grundprinzip der Homöopathie, als das "Gesetz der Ähnlichkeit" bekannt, nämlich "Similia similibus curentur!" also "Ähnliches wird mit Ähnlichem geheilt". Es wurde zuerst von dem deutschen Arzt Samuel Hahnemann im Jahre 1796 angegeben. Homöopathische Mittel werden durch hintereinander geschachtelte Verdünnungen hergestellt, wobei das Schütteln durch kraftvolle markante Schläge auf einem elastischen Körper unterstützt wird. Jede Verdünnung wird durch eine entsprechende Verschüttelung abgeschlossen, um so die Wirkung des pharmazeutischen Mittels zu erhöhen. Diesen Vorgang nennt man Potenzierung. Eine Verdünnung erfolgt oftmals sooft, bis von der ursprünglichen Substanz scheinbar nichts mehr nachgeblieben ist.

Abgesehen von den Symptomen, untersuchen Homöopathen die Aspekte des Patienten in Bezug auf dessen physischen und psychischen Zustand, um im Anschluss mit Hilfe homöopathischer Referenzbücher Abhilfe auf Basis der Gesamtheit der Symptome zu schaffen.

Aus dem Stand der Technik sind unterschiedliche pharmazeutische Mittel und homöopathische Arzneimittel bekannt, um unterschiedliche Wirkungen zu erzielen. Es sind auch pharmazeutische Mittel, insbesondere homöopathische Arzneimittel zur Gewichtsreduzierung bekannt, die dazu beitragen, die Reduktion des Körpergewichtes zu unterstützen. Die bekannten homöopathischen Arzneimittel weisen mehrere Wirkstoffe im Sinne des homöopathischen Wirkstoffbegriffes auf, wobei diese in unterschiedlichen Potenzierungen vorliegen können.
D1 entspricht 1 Teil der ursprünglichen Substanz in 10 Teilen der Lösung,
D2 entspricht 1 Teil der ursprünglichen Substanz in 100 Teilen der Lösung,
D3 entspricht 1 Teil der ursprünglichen Substanz in 1.000 Teilen der Lösung,
D6 entspricht 1 Teil der ursprünglichen Substanz in 1.000.000 Teilen der Lösung,
D9 entspricht 1 Teil der ursprünglichen Substanz in 10⁹ Teilen der Lösung,
D12 entspricht 1 Teil der ursprünglichen Substanz in 10¹² Teilen der Lösung,
D20 entspricht 1 Teil der ursprünglichen Substanz in 10²⁰ Teilen der Lösung,
D23 entspricht 1 Teil der ursprünglichen Substanz in 10²³ Teilen der Lösung.

Die Homöopathie beinhaltet den Prozess der von Homöopathen als "Dynamisierung" oder "Potenzierung" bezeichnet wird. Hierbei wird eine Substanz mit Alkohol oder destilliertem Wasser verdünnt und dann kräftig in einem Prozess der Verschüttelung" hergestellt. Der Begründer der Homöopathie, Samuel Hahnemann glaubte, dass der Prozess der Verschüttelung die "vitale Energie" der verdünnten Substanz aktiviert wird, und dass aufeinanderfolgende Verdünnungen die "Potenz" des Mittels erhöht.

Das homöopathische Arzneibuch (HAB) ist vergleichbar mit anderen pharmazeutischen Büchern und umfasst einen allgemeinen Teil und einen Teil mit Monographien der Ausgangsstoffe, zusätzlich zu ihren üblichen Qualitätsstandards und den Vorgaben. Mehrere Potenz Skalen sind in der Homöopathie bekannt. Homöopathen entwickelte eine Dezimalskala (D oder Dil.), wobei jede Stufe ein Verdünnen der Substanz um das zehnfache seines ursprünglichen Volumens mit sich bringt. Die hintereinander ausgeführte Verdünnung einer Lösung führt nach jeder Verdünnungsstufe dazu, dass weniger Moleküle der ursprünglichen Substanz pro Liter Lösung vorliegen. Homöopathen sind der Auffassung, dass dieses Wasser eine "wesentliche Eigenschaft" des ursprünglichen Materials bewahrt, weil die Zubereitung nach jeder Verdünnung erschüttert worden. Es wird angenommen, dass die Dynamisierung oder Verschüttelung der Lösung eine heilende Kraft mit sich bringt, die sich durch Freisetzungen der Substanzen ergibt. Auch wenn die homöopathischen Mittel oft sehr verdünnt vorliegen, sind Homöopathen sicher, dass eine heilende Kraft von diesen homöopathischen Präparaten ausgeht.

Die Prävalenz der Adipositas (BMI ≥ 30) nimmt in Deutschland seit vielen Jahren kontinuierlich zu. Derzeit sind etwa 50 % der erwachsenen Männer mit einen BMI ≥ 25 übergewichtig und ca. 18% mit einem BMI ≥ 30 adipös. Bei den erwachsenen Frauen sind etwa 35 % übergewichtig und knapp 20 % adipös. Auch bei Kindern und Jugendlichen wurde in den letzten Jahren ein Anstieg beobachtet, so dass hier nunmehr kurzfristig Handlungsbedarf besteht.

Der vorliegenden Erfindung liegt die Aufgabe zugrunde, ein homöopathisches Arzneimittel aufzuzeigen, das es ermöglicht, das Körpergewicht zu reduzieren und langfristig auf einem niedrigeren Niveau zu stabilisieren und zu halten, wobei eine wesentlich höhere Wirksamkeit erzielt werden soll. Insbesondere soll im Körper während der Gewichtsreduzierung ein Wohlbefinden bzw. Glücksgefühl hergestellt bzw. aufrecht erhalten werden. Ferner ist es Aufgabe der Erfindung eine Ernährung für einen Patienten, unter gleichzeitiger Verwendung des homöopathisches Arzneimittels anzugeben, die eine verbesserte Wirkung aufweist. Weiter ist es Aufgabe der Erfindung ein Verfahren zur Gewichtsreduzierung eines Patienten anzugeben, das besonders effizient ist und zudem dem nicht gewollten Jo-Jo-Effekt trotzt.

Gelöst werden diese Aufgaben mit einem homöopathischen Arzneimittel zur Gewichtsreduzierung nach Anspruch 1, eine Ernährung für einen Patienten, unter gleichzeitiger Verwendung des erfindungsgemäßen pharmazeutischen Mittels nach Anspruch 4.

Dadurch, dass das homöopathische Arzneimittel zur Gewichtsreduzierung die folgenden Bestandteile und Komponenten mit den jeweiligen Potenzierungen umfasst, nämlich Adonis vernalis, in D6 Dil., Calcium carbonicum Hahnemann, in D15 Dil., Capsicum, in D6 Dil., Cascara, in D5 Dil., 1 Teil Fucus vesiculosus, D4 Dil., 2 Teile Fucus vesiculosus in D6 Dil., Graphites, in D15 Dil., Kalium Carbonicum, in D8 Dil. und Phytolacca, in D5 Dil., wobei das homöopathische Arzneimittel als wässrige Lösung vorliegt und Natrium-Chlorid in der wässrigen Lösung gelöst ist, ist es erstmalig möglich, gezielt die Reduzierung des Gewichts zu unterstützen bzw. das Körpergewicht zu reduzieren und gleichzeitig das reduzierte Körpergewicht über eine lange Zeit zu halten. Insbesondere werden alle Körperfunktionen positiv beeinflusst und gesteigert. Die körpereigene Fettverbrennung wird durch das Mittel auf natürlicher, pflanzlicher Basis angekurbelt, die Entschlackung des Körpers gefördert und die Bildung von Endorphinen, den Glückshormonen, angeregt. Insbesondere wird der emotionale Hunger unterdrückt, so dass es nunmehr keinerlei Hungergefühle seitens des Patienten gibt, die auf rein emotionaler Ebene entstehen.

Mit der hier vorgestellten Methode, einer neuartigen Methode zur Gewichtsreduktion, die mithilfe einer Ernährungsumstellung und homöopathischen Injektionen arbeitet, erreichen die Teilnehmer sowohl eine Gewichtsabnahme über 10% ihres ursprünglichen Ausgangsgewichtes und ebenfalls eine signifikante Reduktion des BMI, des WHtR und des Taillenumfangs. Dadurch verringern sich auch die mit dem Übergewicht einhergehenden kardiovaskulären Risikofaktoren. Zudem bleiben diese Veränderungen über die Zeit konstant und es tritt kein JoJo-Effekt auf.

Die Behandlung muss mindestens 4 Wochen durchgeführt werden. Es gibt hierfür einen genauen Diätplan, der die richtigen Nährstoffe in errechneter Menge enthält. Bei der Durchführung trifft man sich 3x in der Woche zu Einzelterminen, um gemessen und gewogen zu werden. Bei dieser medizinischen Überwachung werden bei jedem Treffen Injektionen des erfindungsgemäßen homöopathischen Mittels unter die Haut gespritzt, die bewirken, dass sich die Verbrennung/ der Stoffwechsel erhöht, der Körper also mehr Energie, trotz niedriger Kalorienaufnahme verbraucht. Daher soll es recht einfach sein, ohne lästige Quälerei und ohne JoJo-Effekt sein Traumgewicht zu erreichen.

Weitere besondere Vorteile des pharmazeutischen Mittels sind insbesondere eine volle Leistungsfähigkeit, ein vermindertes bzw. völlig fehlendes Hungergefühl, ein "gute Laune" Gefühl durch psychische Stabilität, die Stärkung des Selbstwertgefühls, die Verbesserung des Gesundheitszustandes sowie die Steigerung des Wohlbefindens.

Die Gewichtsreduzierung und die anschließende Gewichtsstabilisierung erfolgt im Wesentlichen durch die Aktivierung des Stoffwechsels mit Hilfe des homöopathischen Arzneimittels. Durch die arzneimittelwirksamen Bestandteile des homöopathischen Arzneimittels erfolgt die Stimulierung zentraler Regulationszentren im Körper. Hierdurch wird der Grundumsatz an Energie erhöht, wodurch der Körper mehr Energie verbraucht.

Zur weiteren Verbesserung sind die Bestandteile in der letzten oder in den letzten beiden Potenzierungen gemeinsam potenziert worden. Insbesondere wird die Wirkung durch das gemeinsame Potenzieren in der letzten bzw. den letzten beiden Potenzierungsschritten gesteigert.

Das homöopathische Arzneimittel weist besonders bevorzugt ausschließlich folgende medizinisch wirksamen Bestandteile in den jeweiligen Potenzierungen auf:
1 Teil Adonis vernalis D6 Dil.,
1 Teil Calcium carbonicum Hahnemann D15 Dil.,
1 Teil Capsicum D6 Dil.,
1 Teil Cascara D5 Dil.,
1 Teil Fucus vesiculosus D4 Dil.,
2 Teile Fucus vesiculosus D6 Dil.,
1 Teil Graphites D15 Dil.,
1 Teil Kalium Carbonicum D8 Dil. und
1 Teil Phytolacca D5 Dil..

Durch diese Kombination von Wirkstoffen fällt es besonders leicht, insbesondere mit der richtigen Kombination von Nährstoffen, die Körpergewichtsreduzierung durchzustehen. Ein Jo-Jo Effekt wird durch das Mittel effektiv verhindert.

Ein weiterer besonderer Vorteil liegt vor, wenn das homöopathische Arzneimittel ausschließlich als wässrige Lösung vorliegt. Es wird gänzlich auf Alkohol als Verdünnungsmittel und/oder Trägermedium verzichtet. Es besteht somit keine alkoholische Basis für dieses homöopathische Arzneimittel. Bevorzugt wird eine Natrium Chlorid-Lösung als Lösung verwendet.

Nach der Reduzierung des Körpergewichts auf ein gewünschtes Gewicht erfolgt eine Nachbehandlung im Anschluss von lediglich vier Wochen, in der das Mittel weiter eingenommen wird.

Ferner ist eine Ernährung für einen Patienten angegeben, wobei diese bei gleichzeitiger Verwendung des homöopathischen Arzneimittels zur Anwendung kommt. Die Ernährung ist ausgeglichen und genau auf die Körperzusammensetzung und das Körpergewicht des Patienten abgestimmt, wobei die Ernährung nur Eiweiße, Kohlenhydrate, Vitalstoffe und Wasser aufweist und besonders bevorzugt aus 22 Prozent Eiweiß, 1 Prozent Kohlenhydrate, 6 Prozent Vitalstoffen und 70 Prozent aus Wasser.

Das offenbarte Verfahren zur Gewichtsreduzierung eines Patienten umfasst die Gabe des homöopathischen Arzneimittels an den Patienten. Hierdurch kann das Körpergewicht in einer sehr hohen und effektiven Weise reduziert werden. Eine Patientin ist in der Lage, ihr Gewicht um ca. 6 bis 8 kg in vier Wochen zu reduzieren. Ein männlicher Patient ist in der Lage sein Körpergewicht um etwa 10 bis 12 Kilogramm in vier Wochen zu reduzieren.

Hierbei erfolgt die Gabe des homöopathischen Arzneimittels mittels eines Applikators durch den Patienten selbst über einen Nadel-freien transmucosalen Applikator zur Injektion in den Mund des Patienten, wobei jedes mal eine definierte Menge des homöopathischen Arzneimittels dem Patienten verabreicht wird. Diese Menge beträgt genau 0,09 ml des homöopathischen Arzneimittels pro Gabe.

Besonders bevorzugt erfolgt die Gabe des homöopathischen Arzneimittels durch den Patienten selbst oder durch den behandelnden Therapeuten oder Arzt mittels subkutaner Injektion, wobei eine Menge von 1,0 ml alle 2 bis 3 Tage, beispielsweise montags, mittwochs und freitags, erfolgt. Hierbei ist besonders vorteilhaft, dass das homöopathische Arzneimittel nicht über die Nahrung zugeführt wird, so dass das homöopathische Arzneimittel nicht im Magen-DarmTrakt unerwünscht zersetzt oder verändert wird.

Besonders vorteilhaft ist es, dass neben der Gabe des homöopathischen Arzneimittels dem Patienten die erfindungsgemäße Ernährung zugeführt wird.

Das gesamte Verfahren wird ausschließlich von geschulten Ärzten oder Heilpraktikern durchgeführt und umfasst nur zwei Stufen:
Stufe 1 - Gewichtsverlust, Stufe 2 - Stabilisierung.

In Stufe 1 (Gewichtsverlust) erfolgt das erste Beratungsgespräch, das dazu dient festzustellen, wie viel Gewicht der Patient verlieren will. Der Patient wird gewogen und gemessen. Danach wird dem Patienten ein Ernährungsplan ausgehändigt. Dieser Ernährungsplan ist einfach und in jeder Situation zu folgen. Dreimal in der Woche findet ein Treffen zwischen dem Patienten und dessen Beratung statt, wobei hier das Gewicht des Patienten kontrollieret wird.

Dem Patienten werden eine exakte Berechnung sowie ein entsprechender Ernährungsplan mit den richtigen Nährstoffen in der jeweils richtigen Menge, zum Beispiel Protein in Form von frischem Fleisch, Kohlenhydrate in Form von frischem Gemüse, Salat und Früchte, ausgehändigt.

Es darf über die Ernährung kein zusätzliches Fett aufgenommen werden, da der Körper mit den körpereigenen Fettreserven auskommen und diese aufzehren soll. Zusätzlich werden Vitalstoffe und Wasser in dem Ernährungsplan angeboten. Die Ernährung für den Patienten ist ausgeglichen und genau auf die Körperzusammensetzung und das Körpergewicht des Patienten berechnet und ausgelegt. Die Ernährung besteht nur aus Eiweiß, Kohlenhydrate, Vitalstoffe wie Vitamine und Mineralien und dergleichen sowie aus Wasser.

Stufe 2 (Stabilisierung) beginnt nach dem Erreichen des gewünschten Gewichts. In dieser Phase wird der Patient von seinem persönlichen Berater dazu gebracht, erkennen zu können, wie er oder sie ihre persönlichen Grenzen in Bezug auf Ernährung erkennt und verwaltet. Der Patient lernt, wie man sein Gewicht mit neuen Essgewohnheiten beibehält. Die Verabreichung des Arzneimittels wird jedoch in jedem Fall fortgesetzt, so dass jeder Patient eine weitere Anwendung des Arzneimittels für nur vier Wochen nach dem Erreichen des vorgesehenen Körpergewichts erhält. In Stufe 2 wird die Dosis des homöopathischen Arzneimittels auf eine Anwendung pro Woche reduziert.

Nach dem Vollenden der Stufe 2, die eine minimale und bevorzugte Zeit von vier Wochen nach der Stabilisierung enthält, ist eine dritte Stufe möglich. Die Patienten können Folgesitzungen buchen, so lange diese notwendig erscheinen. Für beste Ergebnisse wird im Folgenden ein Treffen pro Monat für weitere sechs Monate bevorzugt, um das Gewicht der Patienten zu überprüfen.

Es ist auch möglich, noch mehr Gewicht zu verlieren.

Der beste Weg, dieses hohe Maß an Gesundheit zu halten ist, die Behandlung in regelmäßigen Abständen zu wiederholen, zum Beispiel jedes Jahr im Frühjahr oder Herbst.

Nachfolgend wird nochmals abschließend auf die Besonderheiten eingegangen:
Das Besondere liegt in der Kombination der verschiedenen homöopathischen Wirkstoffe deren Wirkungsweise neu ist. Insbesondere dauerte es 15 Jahre, bis diese komplexe Wirkstoffkombination in müheseliger Kleinstarbeit zusammengestellt werden konnte. Immer wieder sind die homöopathischen Inhaltstoffe und deren Potenzen getestet und geändert bzw. optimiert worden bis schließlich das homöopathische Arzneimittel in der jetzigen Form vorlag.

Nur die Kombination des homöopathischen Arzneimittels löst folgende positive Reaktionen im menschlichen Organismus aus:
- kein JoJo-Effekt;
- durchschnittliche Gewichtsreduktion in 4 Wochen: 6-8 kg;
- Erhaltung und Steigerung der Leistungsfähigkeit;
- Produktion von Endorphinen (Glückshormonen) daher mit guter Laune abnehmen;
- n u r Fettabbau, Muskelmasse wird nicht angegriffen und bleibt konstant
- zusätzliche Heilenergie wird durch Fettabbau freigesetzt
- signifikante Verbesserung des Gesundheitszustandes bei dem metabolischen Syndrom wie z. B.: Diabetes Mellitus Typ II und sogar Typ I, Normalisierung von Bluthochdruck, weniger autoagressive Reaktionen des Immunsystems wie Nahrungsunverträglichkeiten und Allergien
- Verbesserte Leberfunktionen durch Unterstützung der Entgiftung und somit nachgewiesene Normalisierung von Leberwerten (Gamma-GT) im Blut.
- Oft Verbesserung der Hautreaktionen bei Akne, Schuppenflechte, Neurodermitis und keine, wie sonst oft üblich bei größerer Gewichtsreduktion, entstehenden "Fettschürzen" bzw. übrig gebliebene Hautlappen die operativ entfernt werden müssten. Die Haut bleibt straff.

Mittlerweile konnten weitere Tests durchgeführt werden, wobei das durchschnittliche Gewicht der Teilnehmer betrug zu Beginn der Behandlung 96.58 kg und nach 4 Wochen 87.26 kg. Sie haben durchschnittlich 9.32 kg innerhalb dieser 4 Wochen abgenommen, was einem Gewichtsverlust von ca. 2.33 kg in der Woche entspricht. Minimal wurden 3.6 kg, maximal 15.72 kg in diesem 4-Wochenzeitraum abgenommen. Der BMI von 33.25 und der WHtR (Waist to Height Ratio, Taille-zu-Größe-Verhältnis) von 0.62 zu Beginn der Lipoweg-Methode konnte um ca. 10% auf durchschnittlich 30.04 bzw. 0.57 verringert werden. Männer nehmen hierbei im Durchschnitt 0,5 kg in der Woche mehr ab als Frauen. Diese Veränderungen sind hoch signifikant.

Im Durchschnitt nahmen die Teilnehmer während der ersten 4 Wochen mehr als 10% ihres Ausgangsgewichtes ab. Im weiteren Verlauf konnten die Teilnehmer weiterhin abnehmen und dieses Gewicht halten und z.T. noch weiter abnehmen.

Beim Waist-to-height ratio (WHtR) wird berücksichtigt, dass Bauchfett in der Regel negativere Auswirkungen auf die Gesundheit hat als das Fett, welches an anderen Körperregionen sitzt. Auch hier zeigt die Untersuchung, dass der durchschnittliche WHtR von Beginn der Lipoweg-Methode innerhalb der ersten 4 Wochen um mehr als 10% gesunken ist und im weiteren Verlauf weiter fällt. Auch in Phase 3, der Nachbetreuung nach Beendigung der Kur bis zur erneuten Messung ist der WHtR im Durchschnitt weiterhin gesunken (siehe Abbildung 4). Ebenso ist der Taillenumfang von Beginn der Kur bis zum Zeitpunkt t3 über 10% weniger geworden. Dies ist hoch signifikant.

Insgesamt hat die offenbarte Methode nicht die alleinige Gewichtsabnahme und Reduktion des Übergewichts als Folge, sondern reduziert auch die viszerale Fettmasse und reduziert damit augenscheinlich die kardiovaskulären Risikofaktoren.

## Patentansprüche

1. Homöopathisches Arzneimittel zur Verwendung zur Gewichtsreduzierung, umfassend die folgenden medizinisch wirksamen Bestandteile und Komponenten mit den jeweiligen Potenzierungen:
- 1 Teil Adonis vernalis, in D6 Dil.,
- 1 Teil Calcium carbonicum Hahnemann, in D15 Dil.,
- 1 Teil Capsicum, in D6 Dil.,
- 1 Teil Cascara, in D5 Dil.,
- 1 Teil Fucus vesiculosus, D4 Dil.,
- 2 Teile Fucus vesiculosus D6 Dil.,
- 1 Teil Graphites, in D15 Dil.,
- 1 Teil Kalium Carbonicum, in D8 Dil. und
- 1 Teil Phytolacca, in D5 Dil.,
wobei
das homöopathische Arzneimittel als wässrige Lösung vorliegt und Natrium-Chlorid in der wässrigen Lösung gelöst ist.

2. Homöopathisches Arzneimittel zur Verwendung nach Anspruch 1, **dadurch gekennzeichnet, dass** die Bestandteile in der letzten oder in den letzten beiden Potenzierungen gemeinsam potenziert wurden.

3. Homöopathisches Arzneimittel zur Verwendung nach einem der Ansprüche 1 oder 2 zur Verwendung bei der Prävention oder Behandlung der Krankheit Adipositas oder Diabetes Mellitus.

4. Homöopathisches Arzneimittel zur Verwendung nach Anspruch 3 unter gleichzeitiger Verwendung einer ausgeglichenen und genau auf die Körperzusammensetzung und das Körpergewicht des Patienten abgestimmten Ernährung, wobei die Ernährung nur Eiweiße, Kohlenhydrate, Vitalstoffe und Wasser, jedoch kein Fett, aufweist und aus 22 Prozent Eiweiß, 1 Prozent Kohlenhydrate, 6 Prozent Vitalstoffen und 70 Prozent aus Wasser besteht.

5. Homöopathisches Arzneimittel zur Verwendung nach Anspruch 3 oder 4, **dadurch gekennzeichnet, dass** die Gabe des homöopathischen Arzneimittels mittels eines Applikators durch den Patienten selbst über einen Nadel-freien transmucosalen Applikator zur Injektion in den Mund des Patienten erfolgt, wobei jedes mal eine definierte Menge des pharmazeutischen Mittels dem Patienten verabreicht wird.

6. Homöopathisches Arzneimittel zur Verwendung nach einem der Ansprüche 3, 4 oder 5, **dadurch gekennzeichnet, dass** genau eine Menge von 0,09 ml des homöopathischen Arzneimittels pro Gabe dem Patienten zugeführt wird.

7. Homöopathisches Arzneimittel zur Verwendung nach einem der Ansprüche 3 oder 4, **dadurch gekennzeichnet, dass** die Gabe des homöopathischen Arnzeimittels durch den Patienten selbst oder durch den behandelnden Therapeuten oder Arzt mittels subkutaner Injektion erfolgt, wobei eine Menge von 1,0 ml alle 2 bis 3 Tage subkutan injiziert wird.

## Claims

1. A homeopathic medicine for use for weight reduction, comprising the following medically active constituents and components with the respective potentisations:
- 1 part Adonis vernalis, in D6 Dil.,
- 1 part Calcium carbonicum Hahnemann, in D15 Dil.,
- 1 part Capsicum, in D6 Dil.,
- 1 part Cascara, in D5 Dil.,
- 1 part Fucus vesiculosus, in D4 Dil.,
- 2 parts Fucus vesiculosus D6 Dil.,
- 1 part Graphites, in D15 Dil.,
- 1 part Kalium Carbonicum, in D8 Dil. and
- 1 part Phytolacca, in D5 Dil.,
wherein
the homeopathic medicine is present as an aqueous solution and sodium chloride is dissolved in the aqueous solution.

2. The homeopathic medicine for the use according to claim 1, **characterised in that** the constituents are potentised jointly in the last potentisation or in the last two potentisations.

3. The homeopathic medicine for the use according to one of claims 1 or 2 for use in the prevention or treatment of the disease of obesity or diabetes mellitus.

4. The homeopathic medicine for the use according to claim 3 with simultaneous use of a balanced diet adjusted precisely to the body composition and body weight of the patient, wherein the diet comprises only proteins, carbohydrates, vital substances and water, but no fat, and consists of 22 per cent protein, 1 per cent carbohydrates, 6 per cent vital substances and 70 per cent water.

5. The homeopathic medicine for the use according to claim 3 or 4, **characterised in that** the administration of the homeopathic medicine is carried out by the patient him/herself using a needle-free transmucosal applicator for injection into the mouth of the patient, a defined quantity of the pharmaceutical agent being administered to the patient each time.

6. The homeopathic medicine for the use according to one of claims 3, 4 or 5, **characterised in that** a precise quantity of 0.09 ml of the homeopathic medicine is delivered to the patient per administration.

7. The homeopathic medicine for the use according to one of claims 3 or 4, **characterised in that** the administration of the homeopathic medicine is carried out by the patient him/herself or by the treating therapist or doctor by means of subcutaneous injection, a quantity of 1.0 ml being injected subcutaneously every 2 to 3 days.

## Revendications

1. Médicament homéopathique destiné à être utilisé pour la réduction du poids, comprenant les ingrédients et composants médicalement actifs suivants aux dilutions indiquées :
- 1 part d'Adonis vernalis, à la dilution D6
- 1 part de Calcium carbonicum Hahnemanni, à la dilution D15,
- 1 part de Capsicum, à la dilution D6
- 1 part de Cascara, à la dilution D5,
- 1 part de Fucus vesiculosus, à la dilution D4,
- 2 parts de Fucus vesiculosus, à la dilution D6
- 1 part de Graphites, à la dilution D15,
- 1 part de Kalium carbonicum, à la dilution D8, et
- 1 part de Phytolacca, à la dilution D5,
le médicament homéopathique se présentant sous la forme d'une solution aqueuse et du chlorure de sodium étant dilué dans la solution aqueuse.

2. Médicament homéopathique pour une utilisation selon la revendication 1, **caractérisé en ce que** les ingrédients sont dilués ensemble lors de la dernière dilution ou des deux dernières dilutions.

3. Médicament homéopathique pour une utilisation selon la revendication 1 ou 2 pour une utilisation dans la prévention ou le traitement de la maladie adiposité ou du diabète sucré (diabetes mellitus).

4. Médicament homéopathique pour une utilisation selon la revendication 3 avec une utilisation simultanée d'une alimentation équilibrée et adaptée exactement à la composition du corps et au poids corporel du patient, l'alimentation comportant uniquement des protéines, des glucides, des substances vitales et de l'eau, mais pas de graisses, et se composant de 22 pour cent de protéines, 1 pour cent de glucides, 6 pour cent de substances vitales et 70 pour cent d'eau.

5. Médicament homéopathique pour une utilisation selon la revendication 3 ou 4, **caractérisé en ce que** l'administration du médicament homéopathique est réalisée par le patient lui-même au moyen d'un applicateur à l'aide d'un applicateur transmucosale sans aiguille en vue d'une injection dans la bouche du patient, une quantité définie du produit pharmaceutique étant appliquée à chaque fois au patient.

6. Médicament homéopathique pour une utilisation selon l'une des revendications 3, 4 ou 5, **caractérisé en ce qu'**à chaque administration, exactement une quantité de 0,09 ml du médicament homéopathique est appliqué au patient.

7. Médicament homéopathique pour une utilisation selon l'une des revendications 3 ou 4, **caractérisé en ce que** l'administration du médicament homéopathique est réalisée par le patient lui-même ou par le thérapeute soignant ou par le médecin au moyen d'une injection sous-cutanée, une quantité de 1,0 ml étant injectée de façon sous-cutanée tous les 2 à 3 jours.
